# EUROPEAN PATENT APPLICATION

(11) **EP 1 917 925 A1**
(43) Date of publication of application: **07.05.2008**
(21) Application number: 06022835.0
(22) Date of filing: 02.11.2006
(51) Int. Cl.: A61B 17/88, A61B 17/92, A61B 17/16

(54) **Implantation device and implantation device remover and sonotrode for the same**

(71) Applicant: Stryker Trauma GmbH, 24232 Schönkirchen/Kiel (DE)
(72) Inventor: Schwager, Manuel, 4500 Solothurn (DE); Henry, Philip, 2503 Biel/Bienne (CH); Zander, Nils, 24340 Eckenförde (DE); Dorawa, Klaus, 24232 Schönkirchen (DE)
(74) Representative: Haeusler, Rüdiger Hans-Jörg

(57) **Abstract**

According to an exemplary embodiment an implantation device for implantation in a target structure is provided, the implantation device includes a base region, wherein the base region includes a connecting portion, wherein the connecting portion is adapted to interact with a coupling region of a handling device. In particular, the connecting portion and the coupling region may be engageable with each other.

## Description

### Field of the Invention

The invention relates to an implantation device, an implantation kit, an implantation device applicator for the implantation device, a sonotrode for the implantation device and a method for applying the same, in particular a bone pin which is formed as a sonic pin.

### Technical Background

In the prior art several implantation devices for humans or animals are known. The implants at least partly create positive-fit connections to human or animal tissue parts, particularly skeletal parts, wherein the implants help connect tissue parts together, or help connect tissue parts to means supporting or replacing tissue parts, or to other therapeutic auxiliary devices. Further methods for implanting implants into humans or animals are known.

Known implants for creating connections to skeletal parts (bones) include screws, pins, staples, etc., which are used for connecting bones to bones, or bones to artificial, carrying, stabilizing, or supporting parts, or to parts replacing skeletal parts (stabilization or fixation plates, sutures, wires, artificial joint elements, artificial teeth, bone grafts, etc.). Such connection elements for implantation consist for example of metal or plastic, including resorbable plastic. After healing, the connection elements are removed by a further operation or they are left in the body where they are possibly gradually decomposed and replaced by vital tissue.

For stabilizing a bone fracture, a fixation plate with suitable holes is fixed in the region of the fracture using screws as mentioned above. Plate and screws may consist of metal (e.g. stainless steel or titanium). The screws are self-cutting and are rotated into thread less openings in the bone, or they are screwed into pre-drilled threaded openings. Pins are pushed into previously created openings for similar purposes. Connections created in the foregoing manner are usually based on frictional engagement, possibly on positive fit.

It is known also to use curable, plastic materials (e.g. particular cements on a hydraulic or polymer base) for creating connections of the mentioned type. Such materials are pressed from the outside between implant and vital tissue, or into tissue defects in a highly viscous condition, and are cured in situ. Positive-fit connections can be created using such material, if the openings into which the material is pressed comprise suitable undercuts. In order to reduce the stress and/or costs of the corresponding operation method so-called biodegradable implants, e.g. bone pins, are used. That is, bone pins which degrade over time and which are then absorbed by the body. One of such known biodegradable bone pins is known under the trademark Polypin. This bone pin consists of a polyactid-copolymer mixture and is absorbed during a period of about two years.

Also known in the art is the usage of thermoplastic polymer materials which can be liquefied in a targeted manner by way of mechanical oscillation and, in this condition, can be pressed into cavities by way of hydrostatic pressure, thereby creating positive fit connections after solidification.

Such implants may serve for creating positive-fit connections to tissue parts and may consist at least partly of a material that can be liquefied at a relatively low temperature (<250°C) by way of mechanical oscillation energy, such that the material can be pressed into pores or other openings of the tissue part by the effect of external pressure to form positive-fit connections when re-solidified.

For example, from US 6 921 264 an implant for implantation in human or animal bone tissue or in bone tissue supplemented with bone substitute material is known, wherein at least a part of the implant surface comes into contact with the bone tissue, wherein said part of the implant surface comprises surface regions of a first type and surface regions of a second type being different from the surface regions of the first type, wherein the surface regions of the second type comprise a material which is liquefiable by mechanical oscillation and with the aid of which on implantation by mechanical oscillation the implant is stabilized at least primarily in the bone tissue.

For applying the mechanical energy a so-called sonotrode may be used which can be coupled to the implant, e.g. bone pin, and applies the mechanical energy to the bone pin.

A critical factor in the prior art may be that the ultrasonic energy should be efficiently transferred into the implant and in particular efficiently used to liquefy the implant.

### Summary of the Invention

There may be a need to provide an implantation device, an implantation kit, an application device for the implantation device, a sonotrode for the implantation device and a method for applying the same, which implantation device provides for a more efficiently application of mechanical energy in particular when applying ultrasonic energy.

This need may be met by an implantation device, an implantation kit, an application device for the implantation device, a sonotrode for the implantation device and a method for applying the same according to the independent claims.

According to an exemplary embodiment an implantation device for implantation in a target structure is provided, the implantation device includes a base region and a shaft region, wherein the base region includes a connecting portion, wherein the connecting portion is adapted to interact with a coupling region of a handling device and wherein the shaft region comprises a material which is modifiable by using mechanical energy. In particular, the connecting portion and the coupling region may be engageable with each other.

According to an exemplary embodiment a handling device for handling an implantation device according to an exemplary embodiment of the implantation device, wherein the handling device includes a coupling region, which is adapted to couple to the connecting portion of the implantation device.

According to an exemplary embodiment an implantation kit includes an implantation device according to an exemplary embodiment. In particular, the implantation kit may further include a handling device according to an exemplary embodiment.

According to an exemplary embodiment a method for implanting an implantation device according to an exemplary embodiment is provided, the method includes implanting the implantation device into a target structure, and modifying the implantation device by applying mechanical energy. In particular, the modifying may be at least partially done by shearing of parts of the implanted implantation device.

According to an exemplary embodiment an implantation device remover for removing an implantation device from a target structure is provided, wherein the implantation device remover includes a handle region and a drilling region, wherein the drilling region is formed by a hollow shaft having an inner surface, and wherein the inner surface is adapted in such a way that when the implantation device remover is drilled into the target structure by using the handle region, the inner surface cuts a thread into the target surface. The implantation device remover may be in particular adapted to remove an implantation device according to an exemplary embodiment of the implantation device.

According to an exemplary embodiment an implantation kit includes an implantation device remover according to an exemplary embodiment. Preferably, the implantation kit may further include an implantation device, e.g. one implantation device according to an exemplary embodiment, and a handling device, e.g. according to an exemplary embodiment.

According to an exemplary embodiment a method for removing an implantation device is provided which method includes removing the implantation device by using an implantation device remover according to an exemplary embodiment. In particular, the method may further include drilling the implantation device remover into a target structure, in which the implantation device is implanted, in such a way that the drilling region surrounds the implantation device, rupturing the drilled target structure at an distal end with respect to the handle region of the implantation device remover by pulling the handle region of the implantation device remover, and removing the ruptured target structure out of the target structure.

A gist of an exemplary embodiment may be seen in the fact that an implantation device, e.g. a bone pin, is provided which includes a connecting portion which is adapted to interact, e.g. engage, with a coupling region of a handling device, wherein the implantation device includes a shaft region comprising a material which is modifiable by using mechanical energy. In particular, this interaction may include a connecting portion which fits into the coupling region of the handling device. Such a fitting of the two parts may facilitate an improved handling and interaction between the two parts. In particular, the efficiency of application of mechanical energy, in particular when applying ultrasonic energy, may be increased by providing such an interacting interface, e.g. an engaging interface.

In the following, further exemplary embodiments of the implantation device will be described. However, these embodiments apply also for the implantation kit, the application device for the implantation device, a sonotrode for the implantation device and a method for applying the implantation device.

According to another exemplary embodiment of the implantation device the connecting portion is adapted to frictionally interact with the coupling region of the handling device. In particular, the connecting portion may be adapted to be held on the coupling region by friction, even in the case the connecting portion and the coupling region are formed to fit to each other. Thus, an efficient way may be provided to engage the two portions/regions with each other by just sticking the same together, while still a sufficient connecting force is maintained by the friction of the two portions/regions.

According to another exemplary embodiment of the implantation device the connecting portion is formed as a recess. Preferably, the recess is adapted in such a way that the projection of the handling device positively fits into the recess.

By providing a recess as the connection portion of the implantation device it may be possible to provide a suitable guiding and positioning of the two elements relative to each other, i.e. of the implantation device and the handling device. Alternatively, the connection portion may be formed as a protrusion, which is adapted to fit into a corresponding recess of the handling device.

According to another exemplary embodiment of the implantation device the recess has a conical shape. In particular, the conical shape has an angle with respect to a longitudinal axis of the implantation device which angle is adapted to provide self adhesion. Preferably, the angle is less than 10°, in particular less than 8°. Self adhesion may mean that the friction between the two elements is greater than the weight, i.e. the force induced by gravity. An angle of less than 8° may be a suitable angle to provide self adhesion of the implantation device on the handling device. The angle may be measured in a reference system in which 0° denote the case that the wall of the conical shape has the same direction as the longitudinal direction of the implantation device, while 90° denotes the case that the wall is perpendicular to the longitudinal direction of the implantation device. The conical shape may be a real conical shape, i.e. end in a pointed tip, or may be of a trapezoidal shape or truncated conical shape in a longitudinal section, i.e. comprises a flat tip.

According to an aspect of an exemplary embodiment an implantation device for implantation in a target structure is provided, the implantation device comprises a shaft region, wherein the shaft region comprises at least one protrusion, wherein the protrusion comprises a material which is modifiable by using mechanical energy. In particular, the modifiable material may be liquefiable and/or shearable by the mechanical energy, e.g. ultrasonic energy. Preferably, the material may be adapted in such a way that the modifications may be achievable by an energy input which does not destroy human tissue. A suitable frequency of the used ultrasonic energy may be in the range between 28 kHz and 31 kHz. In particular, the frequency may be 29.5 kHz. A suitable amplitude may be in the range between 15 µm and 25 µm. In particular, the amplitude may be about 20 µm. For example, the material may be adapted to exhibit the modification at temperatures below a predetermined temperature threshold. A suitable temperature threshold may be 250°C.

A gist of this aspect of an exemplary embodiment may be seen in that an implantation device is provided including a shaft having at least one protrusion which includes a material which is modifiable by mechanical energy. That is, a implantation device may be provided which includes a protrusion or bump which projects from the substantially smooth shaft region, wherein the shaft region may be circular, elliptical or in the shape of a polygon in cross-section. This protrusion or projection may then be in contact to the surrounding target structure in such a way that when mechanical energy is applied to the implantation device, preferably this protrusion is modified, for example liquefied, melted, or sheared off. The modified material then may act as a bonding agent to bond the implantation device to the surrounding target structure. Since the protrusion projects from the substantially smooth surface of the shaft region of implantation device only a small energy input may be necessary to modify the material of the protrusion. Thus, possibly only a small total input of energy may be necessary in order to bond the implantation device into the target structure, which may lead to the advantage that harmful effects on the surrounding target structure may be omitted or at least reduced. Providing at least one protrusion may further exhibit the advantage that while the implantation device is implanted into the target structure friction is reduced, since only the protrusion may come in contact with the surrounding target material, which may ease the application of the implantation device. The implantation device may be a bone pin or bone plug used to fix a fracture of a bone, for example a human or animal bone. Preferably, the implantation device has a colour which is clearly distinguishable from a colour of the target structure. In particular, the protrusions and/or the implantation device may have blue colour, which may enable a good visibility of the implantation device when the same is implemented into a human or animal body. Preferably, the bone pin is coloured by mixing colour of pigment into the mass the implantation device is made from, e.g. a polymer. Alternatively, the surface of the formed implantation device can be painted by the colour.

According to another exemplary embodiment of the implantation device the modifiable material is bioabsorbable. That is, the material may be absorbed by a human or animals body. Preferably, the bioabsorbable material comprises a copolymer comprising between 50% and 90% Poly-L-lactide and between 10% and 50% Poly-D, L-lactide. In particular, the bioabsorbale material may be a copolymer comprising 70 weight% Poly-L-lactide and 30 weigh% Poly-D, L-lactide. Preferably, the bioabsorbable material may be formed as an amorphous material.

The above described material may be a suitable material for an implantation device, which material may exhibit a suitable tensile strength of about 60 MPa, and a suitable E-modulus of about 3500 MPa. Furthermore, the above material, i.e. an implantation device including the above material, may retain its strength for about a sufficient time when implanted into a human or animals body, Such a time span may be about 16 to 26 weeks. The described copolymer may have a resorption time of about two to three years in a human or animals body. The material may further exhibit an increase of implant volume up to 200% after 24 month from the implantation in the target structure. Such a material may further be easily to be sterilized by y-radiation. A suitable energy dose may be between 20 kGy and 30 kGy, in particular below 25 kGy.

According to another exemplary embodiment of the implantation device the plurality of protrusions are arranged on the shaft in such a way that a flow of liquefied material along the shaft is reduced. Preferably, the shaft has a substantially longitudinal shape and preferably the plurality of the protrusions are arranged displaced, staggered or misaligned to each other along a longitudinal axis of the longitudinal shape. In particular, the plurality of protrusions may be arranged in a pattern which looks like a chess-board pattern, i.e. in a first row of protrusions wherein the protrusion are spaced from each other, while in a next row of protrusions the individual protrusions are placed at the positions of the spaces of the previous row. By arranging the protrusions in such a manner a pattern may be introduced which hinders a flow of the modified or liquefied material along the shaft of the implantation device, i.e. the rows of protrusions may form a dam hindering the flow. By forming such a dam it may be possible to prevent that the liquefied material flows out which may lead to the fact that the bonding may be more secure due to the fact that more liquefied material may solidify after the input of mechanical energy is switched off.

According to another exemplary embodiment of the implantation device the protrusions having a polygonal shape. For example, the protrusions may have a shape of a triangle, a square, trapezoid, or a rectangle. Also circular or elliptical shapes may be suitable. Alternative shapes may be elongated protrusions or tongues, i.e. along the circumference or along the longitudinal axis of the shaft or perpendicular to the longitudinal axis. But also irregular protrusions may be possible. The choosing of the shape may be performed depending on the size of the protrusions and on the possibility to hinder the out flow of liquefied material. A further criterion for the shape may be the ease to shear off parts of the protrusions. In particular, the protrusion may have the polygonal shape in a cross sectional view of the shaft region. Along specific sections of the shaft the cross-section may be identical, but may be different to other, e.g. consecutive sections of the shaft regions. In a side view of the shaft region the individual sections or segments may have a polygonal shape as described above. The different segments, i.e. one segment arranged between two other segments of different cross section, may form channel regions in the shaft region, which channel region may facilitate a main flow of liquefied material in a direction substantially perpendicular to the longitudinal axis of the shaft region, while hindering a flow along the longitudinal.

According to another exemplary embodiment of the implantation device the plurality of protrusions are formed in such a way that during implanting the implantation device into a target material only few protrusions are in contact with the target material, i.e. that not the whole shaft is in contact with the target structure. In particular, the plurality of protrusions may be formed in such a way that during an application of the mechanical energy only a few protrusions are in contact with the target structure. This may lead to the advantage that only at these contact points energy is used to modify, e.g. liquefy, the material so that the total power which may be inputted into the implantation device may be reduceable.

In the following, further exemplary embodiments of the handling device for the implantation device will be described. However, these embodiments apply also for the implantation device, the implantation kit, a sonotrode for the implantation device and a method for applying the implantation device.

According to another exemplary embodiment of the handling device the handling device is formed as an implantation device applicator.

A implantation device applicator may be a device which can be applied for introducing the implantation device into the target structure, e.g. a bone of a human on animal. The use of a specific implantation device applicator to guide the implantation device into the bone may be advantageous compared to a use of an ultrasonic device for guiding and implementing the implantation device, e.g. a bone pin, into the bone, since thus it may be avoidable that the bone pin is already exposed to mechanical or ultrasonic energy while it is introduced into the bone. In particular, the implantation device applicator may comprise a coupling region which is adapted to the shape of the connection portion of the implantation device. For example, the tip of the implantation device applicator may be formed as a cone which can be engaged with a recess which forms the connection portion of the implantation device. The implantation device may be held by frictional force by the conical shape of the tip. By using such a implantation device applicator it may be possible to pick up and hold a cylindrical implantation device securely by engaging the tip of the implantation device applicator and a tapered recess or hole in the base region of the implantation device. In case the implantation device applicator has a diameter of smaller or equal size as the implantation device, e.g. bone pin, itself, such an applicator may be used without drilling a larger hole than necessary for the pin to place it into the bone.

According to another exemplary embodiment of the handling device the implantation device applicator includes an actuating mechanism. In particular, the actuating mechanism may be adapted to decouple the implantation device and the implantation device applicator. Preferably, the actuating mechanism includes a sheath, wherein the sheath is adapted to decouple the implantation device and the implantation device applicator. Furthermore, the actuating mechanism may include an actuating element, wherein the actuating element is adapted to shift the sheath in such a way that it surrounds the coupling region.

By using such an actuating mechanism it may be possible to release the held implantation device at well defined moments. The actuating mechanism may function with an inverse ball point pen mechanism. That is, at pushing the actuating element, e.g. a button, of the actuating mechanism, the implantation device may be peeled off the coupling region of the implantation device applicator, e.g. the cone, by the sheath or a cannulated piece that is pushed over the cone. That is, the pushing of the button shifts the cannulated piece along the longitudinal axis of the implantation device applicator which shifting then decouples the implantation device applicator and the implantation device.

According to another exemplary embodiment of the handling device the handling device is formed as a mechanical energy applicator. In particular, the mechanical energy applicator may be formed as an ultrasound applicator. Preferably, the ultrasound applicator includes a sonotrode having a tip, which tip is adapted to form the coupling region of the handling device, i.e. which is adapted to be coupled to the connecting portion of the implantation device.

According to an exemplary embodiment of the handling device the tip of the sonotrode is further adapted in such a way that the ultrasonic energy is substantially transferred to the base region of the implantation device at areas around the tip. For example, the tip is formed by a conical tip, while around this conical tip a substantially flat region is arranged, i.e. a region which is substantially perpendicular to the longitudinal axis of the handling device. For example, the tip is arranged at the centre of the front face of the handling device, more particularly of the sonotrode of the handling device, and the flat region forms a annular region arranged around the tip. This flat region may interact with a corresponding flat region of the base region of the implantation device and thus provide the basic path for inducing mechanical or ultrasonic energy from the handling device into the implantation device.

According to another exemplary embodiment of the handling device the sonotrode includes a shaft, which has a longitudinal shape, wherein the shaft of the sonotrode includes a coupling element, and wherein the coupling element is adapted to be couplable to a hand piece of the ultrasound applicator in such a way that a moment is transferable from the hand piece to the sonotrode. In particular, the moment may be a torque moment. For example, the coupling element may be formed as a multi-sided profile, e.g. a hexagonal profile .

According to another exemplary aspect of the handling device the coupling element is arranged at a position along the shaft of the sonotrode, which position relates to a node of the ultrasonic wave.

This exemplary aspect may be independent of the above described exemplary embodiments of the handling device, in particular of the embodiments of the sonotrode. In general there may be an inventive concept in the fact that an additional mass concentration of a sonotrode, e.g. a coupling element, is arranged in a node of a stationary or standing wave which is formed in the sonotrode of an ultrasonic device. That is, a sonotrode for a ultrasonic device is provided, wherein the sonotrode comprises additional mass concentration, e.g. a mass which is additional to a generally longitudinal shape of the sonotrode, and wherein the additional mass concentration is arranged in or around a node of the standing wave, i.e. a position exhibit no or at least only very few movement. By providing the extra mass at this very specific location close to the node it may be possible to reduce the influence of the additional mass on the eigenfrequency of the sonotrode. Such an influence may be a crucial factor in the manufacturing of the corresponding sonotrode, since manufacturing tolerances in diameter or other parameter of the sonotrode may have a great influence on the eigenfrequency or resonant frequency of the sonotrode so that the performance of the sonotrode may be greatly reduced or a substantially amount of the sonotrodes has to be rejected for usage. Thus, according to this aspect it may be possible to manufacture sonotrodes less costly and/or in mass-production. Also it may be possible to impose less strict tolerances by manufacturing the sonotrode. The position of the node may be in the region of the middle of the longitudinal extension of the sonotrode. This aspect may be claimed independently or in connection with the above described embodiments of the handling device, in particular of the sonotrode of the handling device.

According to an alternative independent aspect of the invention an implantation device remover for removing an implantation device from a target structure is provided, wherein the implantation device remover includes a handle region, and a drilling region, wherein the drilling region is formed by a hollow shaft having an inner surface, and wherein the inner surface is adapted in such a way that when the implantation device remover is drilled into the target structure by using the handle region the inner surface cuts a thread into the target surface. Such an implantation device remover for removing an implantation device may particularly adapted to remove an implantation device according to an exemplary embodiment of an implantation device described above. For example, it may be possible to provide a coupling region at the implantation device remover which is adapted to couple to the connection portion of the implantation device, thus possibly leading to an efficient guiding of the implantation device remover.

A gist of this alternative aspect may be seen in the fact that a implantation device remover may be provided which uses only a single device for the removing of the implantation device, so that not two different devices as known in the prior art have to be used. In order to achieve this in an efficient way, in an exemplary embodiment the drilling part of the implantation device remover includes an inner surface which is adapted to cut a thread into the target structure, e.g. the bone in which the implantation device may be implemented. By providing this thread a positive locking may be achievable which may lead to the possibility that the cut bone is easily broken at the distal end, i.e. the end which is farther away from the handle of the removing device. In a descriptive way it may be said that the drilling part of the remover builds a cutting hull, cutting wrapper or cutting sheath.

In the following, further exemplary embodiments of the implantation device remover will be described. However, these embodiments apply also for the implantation kit and the method for applying the implantation device remover.

According to another exemplary embodiment of the implantation device remover the drilling region has an end portion, wherein the end portion is formed in such a way that it has a saw like structure.

By providing a saw like end portion an efficient way to enabling the cutting of the target structure, e.g. a bone, may be provided. Thus, by turning the handle region, e.g. a simple T-handle, it may be possible to cut the target structure in an easy way. The turning may be clockwise or counter clockwise. The end region thus may be usable as a kind of crown drill, which cuts the target structure around the implantation device, so that a cylinder like structure is generated including the implantation device and a small amount of target material. This cylinder may then easily be removed from the target structure.

According to another exemplary embodiment of the implantation device remover the cut thread has a pitch which is adapted in such a way that when the implantation device remover is pulled by the handle region out of the target structure the target structure breaks and is removable by the implantation device remover. In particular, the pitch may be a high pitch.

Preferably, the pitch is between 3 mm and 9 mm, more preferably between 5 mm and 7 mm for each turn of the implantation device remover, in case the outer diameter of the drilling region is about 2.5 mm while the inner diameter is about 2.1 mm. This pitch may be lead to the fact that by turning the drilling region by one turn a translation between 3 mm and 9 mm, between 5 mm and 7 mm may be performed. That is, the inclination of the thread may be between 30° and 60° in particular the inclination may be between 40° and 50°, preferably the inclination will be about 45°. In this case the inclination may be defined as the movement along the axis of the drilling region and the corresponding path along the circumferential path along the drilling region.

By providing a thread having such a thread an easy removing of the cylinder which is cut out from the target structure, may be possible.

According to another exemplary embodiment the implantation device remover further includes an ejector unit which is adapted to eject a portion of the target structure which is cut from the target structure. The ejector unit may be formed as a ram or plunger which is adapted to eject the portion of the target structure, e.g. a plug comprising bone and an bone pin implemented into the bone. For providing an easily ejecting of the plug out of the drilling region the pitch or inclination preferably exhibit a low self-locking or self-blocking. While the plug is ejected out of the drilling region the plug may turn around its own axis.

By providing such a unit integrated into the implantation device remover an efficient way for removing the cut target structure out off the remover may be provided. The ejector unit may be in the form of a rod, sheath or hull which can be incorporated into the inner part of the drilling part, e.g. as a portion which can be shifted along an axis of the inner surface of the drilling region inside.

In the following, a further exemplary embodiment of the method for removing a implantation device will be described. However, this embodiment apply also for the implantation device and for the implantation kit.

According to another exemplary embodiment the method further includes drilling the implantation device remover into a target structure, in which the implantation device is implanted, in such a way that the drilling region surrounds the implantation device, rupturing the drilled target structure at an distal end with respect to the implantation device remover by pulling the handle region of the implantation device remover, and removing the ruptured target structure out of the target structure.

It may be seen as the gist of an exemplary embodiment to provide an implantation device remover for removal of a pin from a cancellous bone, in particular of a fused bone pin. For allowing this a core drill with a helical edge on the inside wall of a frontal part of the remover may be provided. The core drill may be adapted to be drilled over the bone pin and the helical edges may fixes the pin by friction or by positive locking. After the remover is drilled into the bone the pin may be broken free distally and pulled out with the same instrument as the hole is drilled. The technique of the remover or instrument could be used for removal of any cylindrical specimen that is distally attached, e.g. bone samples or bone plugs.

Furthermore, it may be seen as the gist of an exemplary embodiment to provide a structure for an implantation device, e.g. a bone pin, which allows to minimize an energy input into the implantation device and thus to a target structure, while still allowing to modifying a state of a material of the implantation device. In order to achieve this the implantation device may comprise a plurality of protrusions projecting from a substantially smooth surface of a shaft of the implantation device. When implanting and fixing this implantation device into the target structure, e.g. broken bone which shall be fixated by the implantation device, mechanical energy like ultrasonic energy is applied to the implantation device leading to a modifying or liquefying of the material of at least the protrusion which liquefied material might be used as a bonding agent promoting a bonding force between the implantation device and the target structure. Due to the small size of the protrusion a contact area between the implantation device and the target structure may be minimized leading to a relatively high energy per area so that grating or melting of the protrusion is promoted although only relatively low energy input and power, i.e. energy per second, is necessary.

According to an exemplary aspect a bone pin may be made of resorbable material, e.g. plastic or polymer, which bone pin features the characteristics that it melts at well defined positions when ultrasound is applied to it. The liquefied polymer may spread out preferably perpendicular to the axis of the bone pin, while spreading out parallel to the axis may be suppressed by fluid barriers which may be formed by protrusions projecting from the surface. Furthermore, a moment of inertia of the bone pin may be substantially equal or at least may not show a great variation along its axis, which may be made possible by a plurality of polygonal protrusions, e.g. in the shape of triangles, which are misaligned relative to each other and which may form a flow barrier. The ultrasonic energy may be concentrated on this certain points on the surface due to the steps formed by the protrusions, e.g. the triangles.

According to the present invention also implantation devices comprising a metal, e.g. titanium, core may be used which have a polymeric overlay, i.e. an overlay comprising a modifiable material. Also bone screws may be provided, in particular bone screws having a metal portion and a tip comprising modifiable material, wherein at the portion of the modifiable material protrusions are formed.

Implantation devices according to an exemplary embodiment may be used for plate fixation in which application the implantation devices, e.g. resorbable pins may be welded to bone and resorbable plate. The use of resorbable pins may provide a fast fixation and a better stability. A further application may be hip fracture fixation. In this application a metal bone screw having a tip of polymeric material, i.e. modifiable material comprising protrusion, may be used. Also this kind of implantation devices may exhibit fast fixation. Further, it might be possible that no rotation of the implantation device is necessary, which rotation possible would displace fragments, due to the fact that the implantation device, e.g. the shaft, does not comprise a thread. Furthermore, such a bone screw having a polymeric tip may be exhibit a better resistance to cut-off.

In general, implantation devices according to an exemplary embodiment may be used in different fields of fixation of bone fragments. In particular, resorbable bone pins may be used in the field of small fragment fixation of foot, ankle, wrist, elbow and shoulder, resorbable mesh/plate and pins may be used in the field of small fragment fixation of ankle, wrist, and graft containment and hybrid metal and resorbable pins may be used in the field of distal locking, bone anchor, femoral neck fractures, trochaneric fractures, and ex fix pin fixation in osteoporotic bones.

These and other aspects of the present invention will become apparent from and elucidated with reference to the embodiment described hereinafter.

### Brief Description of the Drawings

Exemplary embodiments of the present invention will be described in the following, with reference to the following drawings.
Fig. 1A shows a schematic illustration of a bone pin according to an exemplary embodiment;
Figs. 1B to 11 are showing enlarged views of the bone pin of Fig. 1A;
Fig. 2 shows a schematic illustration of a base region of an implantation device and a corresponding sonotrode tip according to an exemplary embodiment of the invention;
Figs. 3A and 3B show a schematic illustration of a sonotrode according to an exemplary embodiment of the invention;
Fig. 3C shows a schematic illustration of an ultrasonic device for a sonotrode;
Fig. 4 shows a schematic illustration of a base region of an implantation device and a corresponding implantation device applicator tip according to an exemplary embodiment of the invention;
Figs. 5A to 5D show schematic illustrations of an implantation device applicator according to an exemplary embodiment of the invention; and
Figs 6A to 6C show schematic illustration of an implantation device remover according to an exemplary embodiment of the invention.

### Detailed Description of Exemplary Embodiments

The illustration in the drawings is schematically. In different drawings, similar or identical elements are provided with similar or identical reference signs.

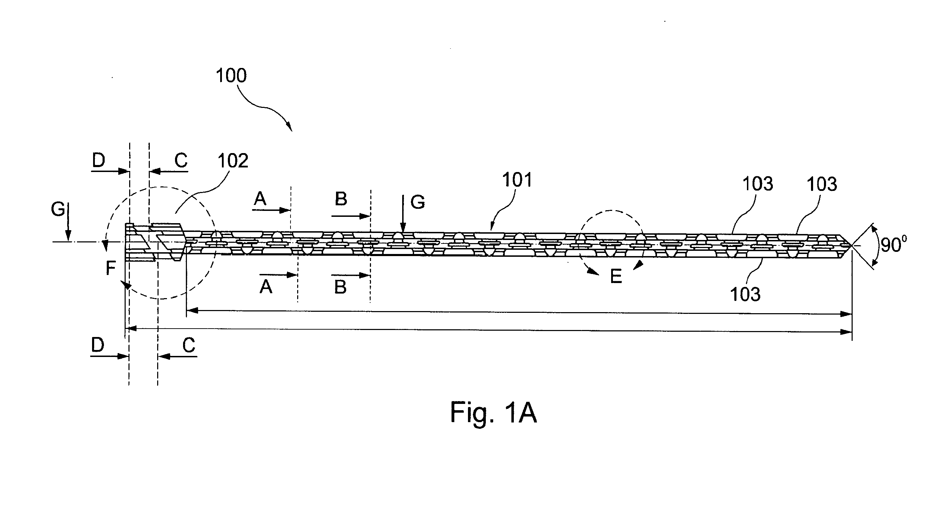

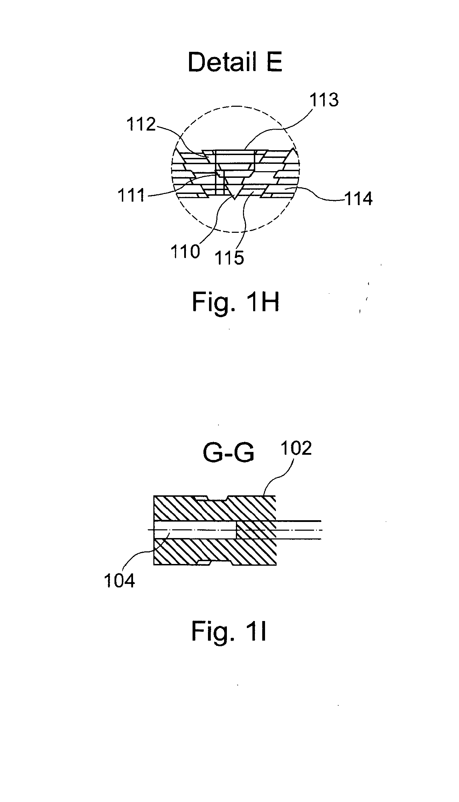

Fig. 1A shows a schematic illustration of the bone pin 100 according to an exemplary embodiment. The bone pin 100 comprises a shaft region 101 and a base region 102. The shaft region 101 comprises a plurality of protrusions 103 which roughly have the general shape of a triangle. A single one of the protrusions is depicted in greater detail in Fig. 1H. The base region 102 may be adapted to fit onto a corresponding ultrasonic device.

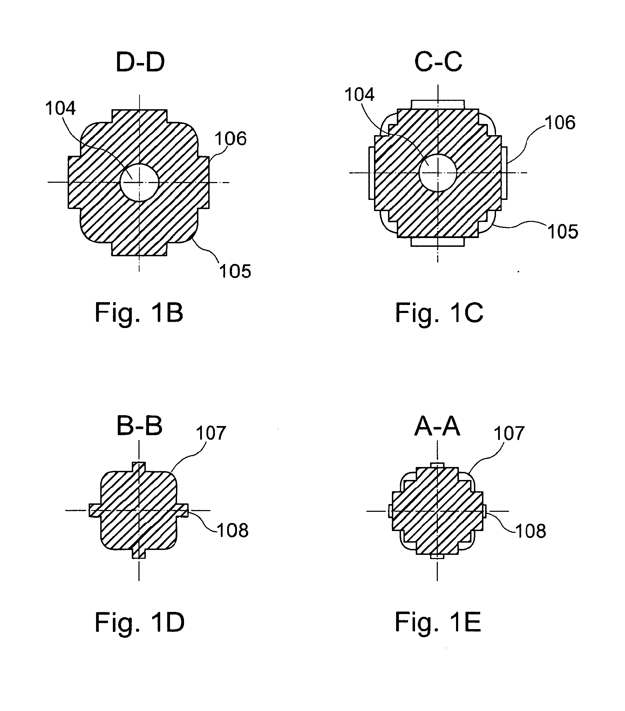

Fig. 1B shows a enlarged view of the sonic pin 100 of Fig. 1A. In particular, it shows a cross section along line D-D of Fig. 1A, i.e. a cross section of the base region 102. In the centre of the cross section of Fig. 1B a hole 104 is shown which might be adapted to accommodate a portion of the ultrasonic device, e.g. a sonotrode of the ultrasonic device. Furthermore, it can be seen in Fig. 1B that the cross section comprises rounded regions 105 and substantially plane regions 106.

Fig. 1C shows another enlaregd view of the sonic pin 100 of Fig. 1A. In particular, it shows a cross section along line C-C of Fig. 1A, i.e. also a cross section of the base region 102. In the centre of the cross section of Fig. 1C the hole 104 is shown. Furthermore, in Fig. 1C the rounded regions 105 and the substantially plane regions 106 can be seen.

Fig. 1D shows another enlarged view of the sonic pin 100 of Fig. 1A. In particular, it shows a cross section along line B-B of Fig. 1A, i.e. a cross section of the shaft region 101. Also this cross section shows further rounded regions 107 and further substantially plane regions 108. In particular, the further plane regions 108 may be part of the protrusions 103.

Fig. 1E shows another enlarged view of the sonic pin 100 of Fig. 1A. In particular, it shows a cross section along line A-A of Fig. 1A, i.e. a cross section of the shaft region 101. Also this cross section shows the further rounded regions 107 and the further substantially plane regions 108.

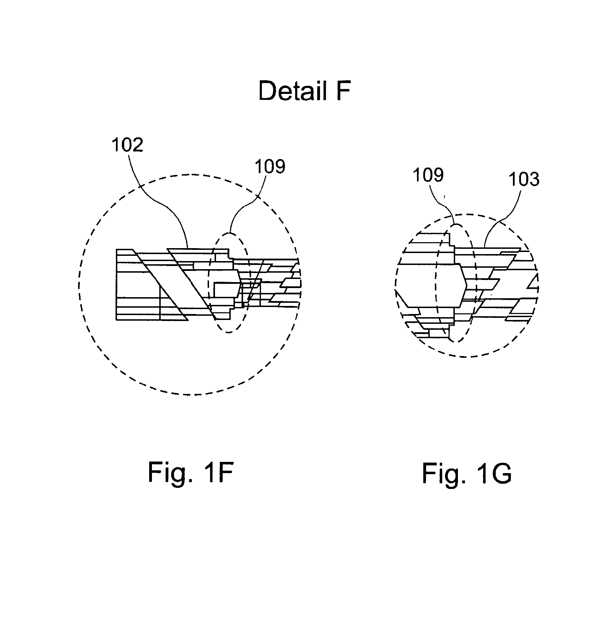

Fig. 1F shows another enlarged view of the sonic pin 100 of Fig. 1A. In particular, it shows a detailed view of the portion which corresponds to the circle labeled F in Fig. 1A, i.e. of the base region 102. In particular, Fig. 1F shows the transition portion 109 between the base region and the shaft region.

Fig. 1G shows an even more enlarged view of the portion of Fig. 1F, in which the transition region can be seen more clearly. In particular Fig. 1G also shows a part of a protrusion 103 in more detail. The protrusions of the embodiment shown in Fig. 1 have an overall shape, which is roughly triangular. The single triangles are formed by tongues 110, 110 and 112 which do have a different length along the axis of the sonic pin 100. Due to the different lengths a roughly triangular shape of the protrusions results. Such a shape may be suitable to hinder the flowing of liquefied material along the axis of the sonic pin 100 and promote a more perpendicular spreading of the liquefied material, i.e. a spreading which promote the flowing of the liquefied material into cavities or pores which are formed in bone material of human or animal bones.

Fig. 1H shows another enlaregd view of the sonic pin 100 of Fig. 1A. In particular, it shows a detailed view of the portion which corresponds to the circle labeled E in Fig. 1A, i.e. a protrusion formed in the shaft region 101. As well as in Fig. 1G the roughly triangular shape of the protrusion can be seen in Fig. 1H, which shape is formed by the tongues 110, 111, and 112 having different lengths. Furthermore, it can be seen in Fig. 1H that the orientation of the triangular protrusion 103 are altering, i.e. after a first triangular protrusion 113 having its base length in Fig. 1H at the upper side, a consecutive triangular protrusion 114 has its base length at the bottom side, leading to channel regions 115 between the single triangles, which channels 115 may be suitable to direct the flow of liquefied material.

Fig. 11 shows another enlarged view of the sonic pin 100 of Fig. 1A. In particular, it shows a detailed cross sectional view along line G-G in Fig. 1A, i.e. a cross sectional view of the base region 102. In particular, the hole 104 in the base region can be seen, in which a tip of a sonotrode or an implantation device applicator may be inserted.

A bone pin according to an exemplary embodiment may be used in a operation process for fixation of a broken or splintered bone, the procedure comprising the following steps. Drilling a hole in which the bone which has to be fixed afterwards the bone pin is implemented into the drilled hole. In case the bone is a porous bone, i.e. comprising porous material it may not necessary to pre drill a hole. Preferably, this is done using an so-called pin applicator. Afterwards the pin applicator is removed and an ultrasonic device is arranged at the base region of the bone pin, i.e. in the hole which is shown as 104 in Fig. 1. When the ultrasonic device is switched on the bone pin starts to oscillate and the protrusions arranged on the shaft of the bone pin come into contact with the surrounding bone so that portions of the protrusions are sheared off by shearing forces from the bone pin, whereby they are liquefied. Due to the providing of a plurality of protrusion comprising tongues only small parts of the hole shaft of the bone pin are contacting the bone and are sheared off or liquefied. The liquefied material of the bone pins starts to penetrate pore spaces of the bone. When the ultrasonic device or ultrasonic driver is switched off the liquefied material, e.g. the polymer, cools rapidly, resulting in a stable joint after only a few seconds and may form a positive locking. In case the bone pin should be removed again a bone pin remover may be used. Such a pin remover may comprise a portion similar a crown drill, which drill is placed above the bone pin which is to be removed. Then the remover is drilled into the bone. The cutting hull or cutting sheath of the crown drill cuts into the bone and also cuts a thread into the bone cut in the cutting hull. This threading may provide a positive locking between the driller and the bone portion to be removed so that afterwards the remover may be used to break the bone portion including the bone pin to be removed.

The above described bone pine, according to an exemplary embodiment of the invention, has a surface which is rougher than the surface of known bone pins. This roughness may be caused by the plurality of protrusions which may also be called energy raisers. For testing two different types of bone pins, i.e. one having a rough surface according to an exemplary embodiment of the invention while the other one having a smooth surface as known in the prior art, are inserted in bone substitute material. The energy input needed to insert the pins to the same depth was measured. In the testing a clear difference in energy input needed to insert the bone pin arose. For a bone pin according to an exemplary embodiment of the invention, i.e. a rough bone pin, about 19% less energy input is needed when compared to a known, i.e. smooth bone pin. In particular, the mean energy input needed for the known bone pin was about 58.6 J, while for a rough bone pin, i.e. a bone pin having a plurality of protrusion, the mean energy input was about 47.3 J. Also the fusion time was reduced from about 2.5 s to about 2 s in the case of a bone pin according to an exemplary embodiment of the invention was used.

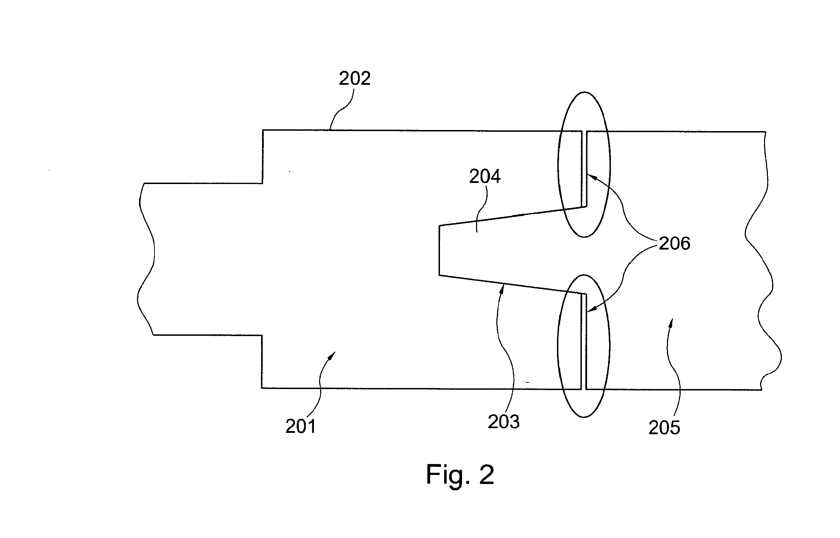

Fig. 2 shows a schematic illustration of a base region of an implantation device and a corresponding sonotrode tip according to an exemplary embodiment of the invention. In detail Fig. 2 shows a base region 201 of an implantation device 202. The base region 201 includes a partly conical recess 203 formed in the base region. Furthermore, a tip 204 of a sonotrode 205 is shown in Fig. 2. The tip 204 of the sonotrode 205 has a shape which engages with the recess 203 of the base of the implantation device. An angle of the conical shape with respect to a longitudinal axis of the implantation device and the sonotrode is preferably less than 10°, which small angle may result in a self adhesion of the base region of the implantation device on the sonotrode tip. That is, when such a small angle is used, the implantation device, e.g. a bone pin, is held through friction by the cone of the sonotrode tip, which may lead to the fact that a good guidance and positioning of the implantation device with respect to the sonotrode tip. The transferring of the mechanical energy, e.g. ultrasonic energy, from the sonotrode tip to the implantation device is substantially provided by the end faces of the sonotrode tip, i.e. the areas which are labelled 206 in Fig. 2. Preferably, the sonotrode tip is manufactured from one piece, i.e. integrally formed with the sonotrode itself and does not form a part which is separately formed and then bonded to the sonotrode.

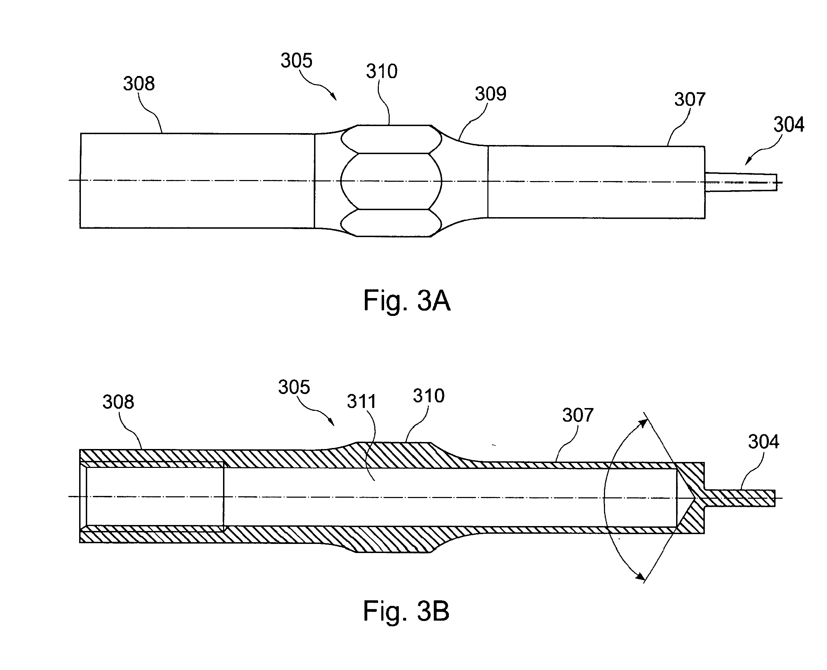

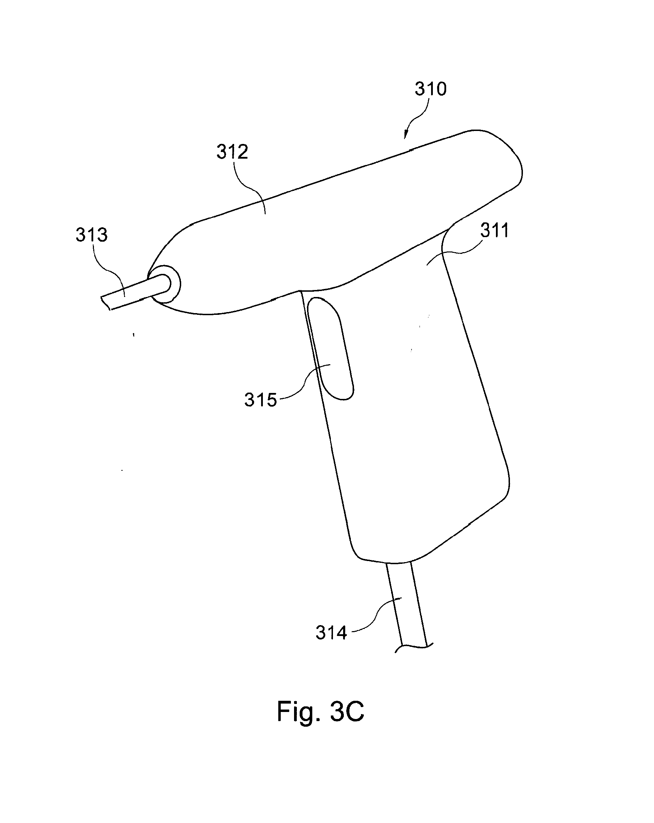

Figs. 3A and 3B show a schematic illustration of a sonotrode according to an exemplary embodiment of the invention. Fig. 3A shows a sonotrode 305 comprising a tip 304 having at least in part a conical shape as already shown in Fig. 2. Furthermore, the sonotrode 305 comprises a front region 307 of substantially cylindrical shape and a rear region 308 of basically cylindrical shape as well. Between the front and the rear regions a centre region 309 is arranged comprising a coupling element 310. In the embodiment shown in Fig. 3 the coupling element is of basically polygonal shape, e.g. of hexagonal shape, but may be of any other suitable shape. The coupling region 309 primarily serves to couple the sonotrode to a hand piece of an ultrasonic device in which the sonotrode is fitted. By using a polygonal shape a moment, e.g. a torque moment, can be transferred from the ultrasonic device to the sonotrode. This torque moment may also, at least to some extent transferable to an implantation device in case this implantation device is engaged with the sonotrode tip.

The coupling element 310 is arranged in a node of a stationary wave used to transfer the mechanical energy from the ultrasonic device to the implantation device. By arranging the coupling element, or any other additional mass concentration, in this specific point, which is roughly in the middle of the sonotrode, of the sonotrode it may be possible to eliminate or at least reduce the influence of this additional mass on the eigenfrequency of the sonotrode. The eigenfrequency or resonant frequency of the sonotrode has to be adapted to the eigenfrequency of the hand piece to a rather great extent, i.e. has to be adapted quite well. Therefore, the variations in the manufacturing of the sonotrode do have a great influence on the function of the sonotrode and on the waste sonotrodes which has to be separated out. Since according to this embodiment the additional amount of mass is positioned in a node of the ultrasonic wave a greater variations in manufacturing of this additional mass can be tolerated which may lead to reduced production costs.

Fig. 3B shows a longitudinal section of Fig. 3A and shows the sonotrode 305, the tip 304, the front region 307, and the rear region 308 of Fig. 3A as well. Further, the centre region 309 comprising the coupling element 310 can be seen. Furthermore, it can be seen in Fig. 3B that the sonotrode may be hollow, i.e. may be formed substantially of a hollow cylinder comprising a region 311 around its axis in which no material exists. Preferably, the front region of this hollow space, i.e. the region which is close to the tip of the sonotrode, has a conical shape, wherein the angle of the corresponding cone is greater than 90°, preferably about 120°, e.g. 118°.

Fig. 3C show a schematic illustration of an ultrasonic device 310 which can be used in combination with a bone pin according to an exemplary embodiment of the invention. The ultrasonic device comprises a hand piece 311 which has a shape which is adapted to be gripped by a human, e.g. a physician implementing the bone pin. Furthermore, the ultrasonic device comprises a mounting part 312 in which a sonotrode 313 can be inserted. Furthermore, a cable 314 for supplying the ultrasonic device with energy and a switch 315 for turning the ultrasonic device on and off is shown in Fig. 3C.

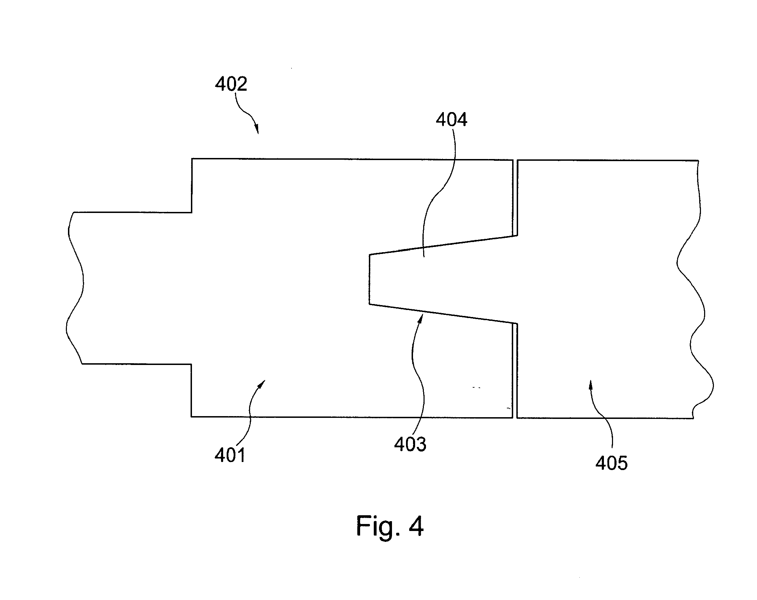

Fig. 4 shows a schematic illustration of a base region of an implantation device and a corresponding implantation device applicator tip according to an exemplary embodiment of the invention. In detail Fig. 4 shows a base region 401 of an implantation device 402. The base region 401 includes a conical recess 403 formed in the base region thereof. Furthermore, a tip 404 of an implantation device applicator 405 is shown in Fig. 4. The tip 404 of the implantation device applicator 405 has a shape which engages with the recess 403 of the base of the implantation device. An angle of the conical shape with respect to an longitudinal axis of the implantation device and the applicator is preferably less than 10°, which small angle may result in a self adhesion of the base region of the implantation device on the applicator tip. That is, when such a small angle is used, the implantation device, e.g. a bone pin, is held through friction by the cone of the applicator tip.

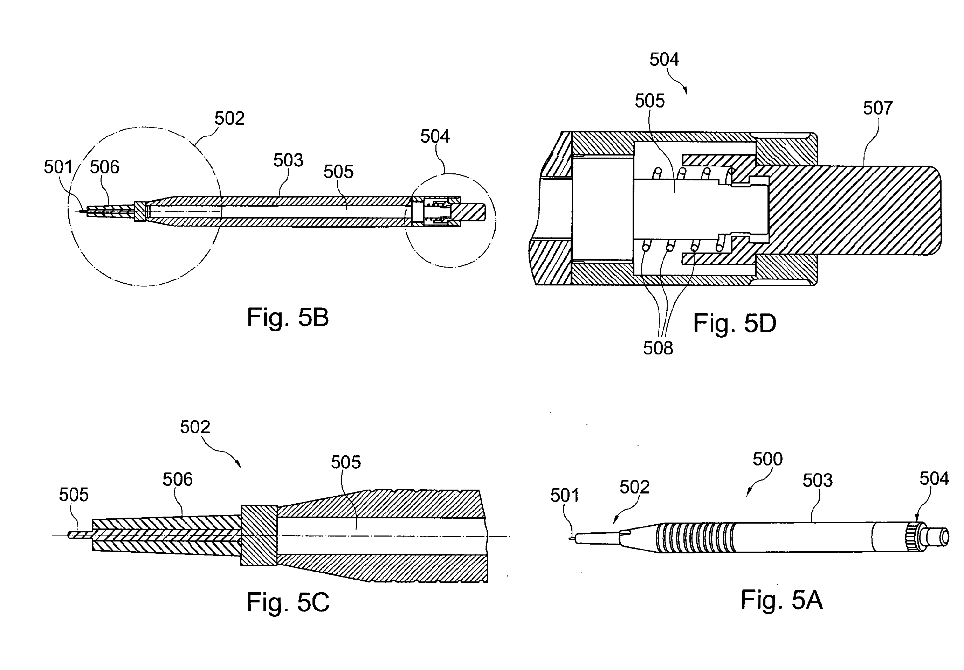

Figs. 5A to 5D show schematic illustrations of an implantation device applicator 500 according to an exemplary embodiment of the invention. Fig. 5A shows a perspective view of the implantation device applicator 500. The implantation device applicator 500 comprises a tip 501 and a front region 502. Furthermore, the implantation device applicator 500 comprises a main body 503 and an end region 504. The tip, the front region and the end region are depicted in greater detail in Figs. 5C and 5D.

Fig. 5B shows a sectional section along the longitudinal axis of the implantation device applicator 500. The main body 503 has a substantially cylindrical hollow shape which can accommodate an actuation element 505. The actuating element may consists of a rod which might be used to actuate a hull, barrel or sheath 506. When actuating the actuating element and thus shifting the hull 506, the hull may be slide along the longitudinal axis of the implantation device applicator 500 in such a way that the tip 501 is enclosed by the hull 506 leading to the effect that an implantation device engaged with the tip of the implantation device applicator 500 will be disengaged from the tip of the implantation device applicator 500. The actuation element 505 may be activated by a mechanism similar to the mechanism of a ball point pen mechanism, e.g. an inverse ball point pen mechanism.

Fig. 5C shows the tip of implantation device applicator 500 in greater detail and corresponds to the front region 502 encircled by the first circle in Fig. 5B. In Fig. 5C the tip 501, the hull 506 the main body 503 and the actuation element 505 can be clearly seen.

Fig. 5D shows the end or rear region 504 of the implantation device applicator 500 in greater detail and corresponds to the end region encircled by the second circle in Fig. 5B. In particular, an actuation mechanism is shown in greater detail. The actuation mechanism comprises a button 507 similar to a button of a ball point pen. The actuation mechanism further comprises an elastic element, e.g. a spring 508, which engages into a recess of the button and serves to push the button 507 back into a rest position, in which the button is moved away from the tip of the implantation device applicator. When the button 507 is pressed, the button is moved in the longitudinal direction towards the tip of the implantation device applicator which causes the actuation element 505, which also engages into the recess of the button, to be moved towards the tip. The movement of the actuation element 505 in turn causes that the hull as well is moved towards the tip, so that an implantation device engaged with the tip of the implantation device applicator will be disengaged.

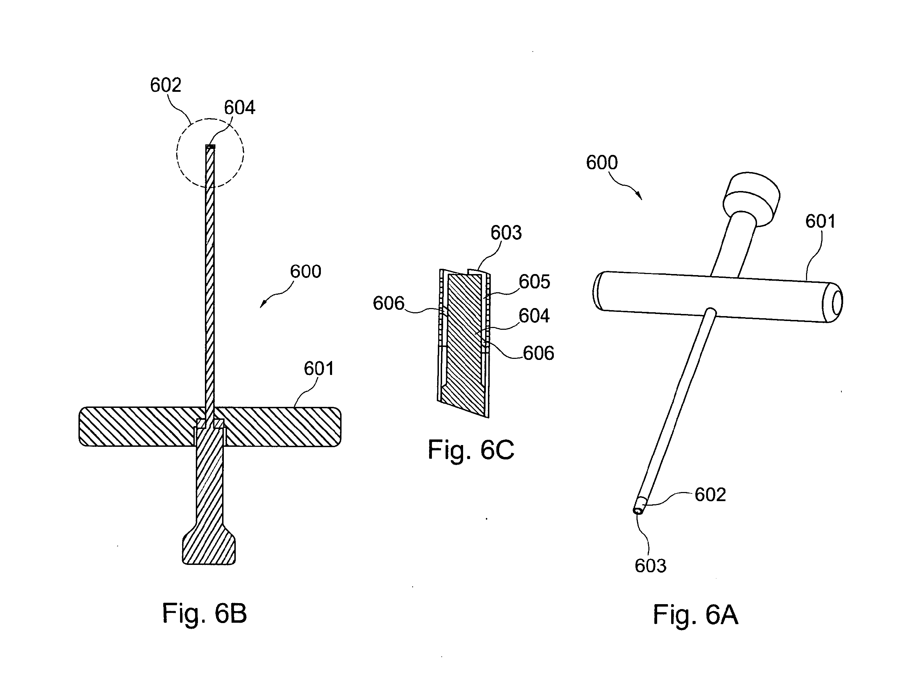

Fig. 6A shows a schematic view of an implantation device remover 600 according to an exemplary embodiment. The implantation device remover 600 comprises a handle 601, which is in the case of Fig. 6 a simple T-piece. The handle 601 serves for a good grip of a person using the implantation device remover, e.g. a surgeon. The implantation device remover 600 further comprises a drilling region 602 which comprises at a tip of the drilling region 602 a hollow part. Furthermore, the tip comprises an edge 603 which is formed like a saw, e.g. comprises cutting teeth. In total the implantation device remover 600 has a size which is suitable for good gripping by the surgeon, e.g. the handle is about 100 mm wide. The diameter of the drilling region 602 is adapted to the diameter of the implantation device which is to be removed. In particular, the inner diameter of the hollow part may be a little greater than the diameter of the implantation device.

Fig. 6B shows a schematically longitudinal cut through the implantation device remover of Fig. 6A. Furthermore, the implantation device remover 600 comprises an ejector unit 604 which is more clearly visible in Fig. 6C. The ejector unit 604 is slidable mounted in the inner part of the drilling region.

Fig. 6C shows a detailed view of the tip of the implantation device remover of Fig. 6B.In particular, in the detailed view of Fig. 6C the cutting edge 603 is clearly visible. Further, a the inner surface 605 of the drilling part is shown on which a helical edge 606 is schematically shown, which serves to cut a thread into the bone. Furthermore, the ejection unit 604 can be seen in Fig. 6C more clearly, which is in case of the shown embodiment a rod like structure. When the drilling region is drilled into the bone, the ejector unit 604 is pushed up the longitudinal axis of the implantation device remover. After the bone piece to be removed and including the bone pin has been drilled out and has been broken out of the bone the ejector unit 604 may be used to remove the cylindrical bone piece out of the drilling region implantation device remover 600.

In general a bone pin may be used in a operation process for fixture of a broken or splintered bone, the procedure comprising the following steps. Drilling or pre-drilling a hole into the bone which has to be fixed, afterwards the bone pin is implemented into the drilled hole. The pre-drilling hole may act as a guidance for the bone pin. Preferably, the pre-drilling is performed in such a way that all fragments of the broken or splintered bone are penetrated, so that all fragments are contactable by the bone pin in such a way that all fragments are joinable. Preferably, this is done using an so-called pin applicator. Afterwards the pin applicator is removed and an ultrasonic device is arranged at the base region of the bone pin. When the ultrasonic device is switched on the bone pin starts to oscillate and the protrusions arranged on the shaft of the bone pin come into contact with the surrounding bone so that portions of the protrusions are sheared off by shearing forces from the bone pin, whereby they are liquefied. Due to the providing of a plurality of protrusion comprising burrs or ridges only small parts of the hole shaft of the bone pin are contacting the bone and are sheared off or liquefied. The liquefied material of the bone pins starts to penetrate pore spaces of the bone. When the ultrasonic device or ultrasonic driver is switched off the liquefied material, e.g. the polymer, cools rapidly, resulting in a stable joint after only a few seconds and may form a positive locking. In case the bone pin should be removed again the bone pin remover according to an exemplary embodiment of the invention may be used. Such a pin remover may comprise a portion similar a crown drill, which driller is placed above the bone pin which is to be removed. Then the remover is drilled into the bone. The cutting hull of the crown drill cuts into the bone and also cuts a thread into the bone cut in the cutting hull. This threading may provide a positive locking between the driller and the bone portion to be removed so that afterwards the remover may be used to break the bone portion including the bone pin to be removed.

Summarizing it may be seen as one aspect of an exemplary embodiment of the present invention to provide a bone pin having a shaft comprising a plurality of protrusions. Due to the protrusions only small parts of the bone pins are sheared off when ultrasonic energy is applied to the bone pin, leading to the effect that only a small total power is used to achieve a suitable energy density at the protrusions to liquefy the same. Thus, the total power, i.e. energy per time, may be reduced which may reduce the energy input into the bone the bone pin is applied to, so that damage to the bone may be reduced. Furthermore, the implantation device includes a base region having a recess formed therein which is adapted to accommodate a tip of a handling device or an implantation device remover.

It should be noted that the terms "comprising" or "including" do not exclude other elements or steps and the "a" or "an" does not exclude a plurality. Also elements described in association with different embodiments and aspects may be combined. It should also be noted that reference signs in the claims shall not be construed as limiting the scope of the claims.

## Claims

1. An implantation device for implantation in a target structure, the implantation device comprising:
a base region; and
a shaft region
wherein the base region comprises a connecting portion,
wherein the connecting portion is adapted to interact with a coupling region of a handling device, and
wherein the shaft region comprises a material which is modifiable by using mechanical energy.

2. The implantation device according claim 1,
wherein the connecting portion is adapted to frictionally interact with the coupling region of the handling device.

3. The implantation device according claim 1 or 2,
wherein the connecting portion is formed as a recess.

4. The implantation device according claim 3,
wherein the recess is adapted in such a way that the projection of the handling device positively fits into the recess.

5. The implantation device according claim 3 or 4,
wherein the recess has a conical shape.

6. The implantation device according to claim 5,
wherein the conical shape has an angle with respect to a longitudinal axis of the implantation device which angle is adapted to provide self adhesion.

7. The implantation device according claim 6,
wherein the angle is less than 10°.

8. The implantation device according anyone of the preceding claims, wherein the shaft region comprises a at least one protrusion;
wherein the protrusion comprises a material which is modifiable by using mechanical energy.

9. The implantation device according claim 8,
wherein the modifiable material is bioabsorbable.

10. The implantation device according claim 8 or 9, further comprising:
a plurality of protrusions,
wherein the plurality of protrusions are arranged on the shaft region in such a way that a flow of liquefied material along the shaft is reduced.

11. A handling device for handling an implantation device according to anyone of the claims 1 to 10,
wherein the handling device comprises a coupling region, which is adapted to couple to the connecting portion of the implantation device.

12. The handling device according to claim 11,
wherein the handling device is formed as an implantation device applicator.

13. The handling device according claim 12,
wherein the implantation device applicator comprises an actuating mechanism.

14. The handling device according claim 13,
wherein the actuating mechanism is adapted to decouple the implantation device and the implantation device applicator.

15. The handling device according claim 14,
wherein the actuating mechanism comprises a sheath,
wherein the sheath is adapted to decouple the implantation device and the implantation device applicator.

16. The handling device according claim 15,
wherein the actuating mechanism comprises an actuating element,
wherein the actuating element is adapted to shift the sheath in such a way that it surrounds the coupling region.

17. The handling device according to claim 11,
wherein the handling device is formed as a mechanical energy applicator.

18. The handling device according claim 17,
wherein the mechanical energy applicator is formed as an ultrasound applicator.

19. The handling device according claim 18,
wherein the ultrasound applicator comprises a sonotrode having a tip, which tip is adapted to form the coupling region of the handling.

20. The handling device according claim 19,
wherein the tip of the sonotrode is further adapted in such a way that the ultrasonic energy is substantially transferred to the base region of the implantation device at areas around the tip.

21. The handling device according claim 19 or 20,
wherein the sonotrode comprises a shaft, which has a longitudinal shape,
wherein the shaft of the sonotrode comprises a coupling element, and
wherein the coupling element is adapted to be couplable to a hand piece of the ultrasound applicator, in particular in such a way that a moment is transferable from the hand piece to the sonotrode.

22. The handling device according to claim 21,
wherein the moment is a torque moment.

23. The handling device according claim 21 or 22,
wherein the coupling element is formed as a multi-sided profile.

24. The handling device according to anyone of the claims 21 to 23,
wherein the coupling element is arranged at a position along the shaft of the sonotrode, which position relates to a node of the ultrasonic wave.

25. An implantation kit comprising:
an implantation device according to anyone of the claims 1 to 10.

26. The implantation kit according claim 25, further comprising:
a handling device according to anyone of the claims 11 to 24;

27. A method for implanting an implantation device according to anyone of the claims 1 to 10, the method comprising:
implanting the implantation device into a target structure; and
modifying the implantation device by applying mechanical energy.

28. The method according to claim 27,
wherein the modifying is partially done by shearing of parts of the implanted implantation device.

29. An implantation device remover for removing an implantation device from a target structure, the implantation device remover comprising:
a handle region;
a drilling region,
wherein the drilling region is formed by a hollow shaft having an inner surface,
wherein the inner surface is adapted in such a way that when the implantation device remover is drilled into the target structure by using the handle region the inner surface cuts a thread into the target surface.

30. The implantation device remover according claim 29,
wherein the drilling region has an end portion, and
wherein the end portion is formed in such a way that it has a saw like structure.

31. The implantation device remover according to claim 29 or 30,
wherein the cut thread has a pitch which is adapted in such a way that when the implantation device remover is pulled by the handle region out of the target structure the target structure breaks and is removeable by the implantation device remover.

32. The implantation device remover according claim 31,
wherein the pitch is a high pitch.

33. The implantation device remover according claim 31 or 32,
wherein the pitch is substantially 1: ??

34. The implantation device remover according anyone of the claims 29 to 33, further comprising:
an ejector unit,
wherein the ejector unit is adapted to eject a portion of the target structure which is cut from the target structure.

35. An implantation kit comprising:
an implantation device remover according to anyone of the claims 29 to 34.

36. The implantation kit according claim 35, further comprising:
a handling device according to anyone of the claims 11 to 24;
an implantation device according to anyone of the claims 1 o 10; and
an ultrasonic device.

37. A method for removing an implantation device, the method comprising:
removing the implantation device by using an implantation device remover according to anyone of the claims 29 to 34.

38. The method according claim 37, further comprising:
drilling the implantation device remover into a target structure, in which the implantation device is implanted, in such a way that the drilling region surrounds the implantation device;
rupturing the drilled target structure at an distal end with respect to the handle region of the implantation device remover by pulling the handle region of the implantation device remover; and
removing the ruptured target structure out of the target structure.
